# EUROPEAN PATENT SPECIFICATION

(11) **EP 2 346 496 B1**
(45) Date of publication and mention of the grant of the patent: **02.07.2014**
(21) Application number: 09820892.9
(22) Date of filing: 14.10.2009
(51) Int. Cl.: A61K 9/28, B05D 1/02, B08B 5/02, C23C 24/00, A61J 3/00, B07B 4/00, B05D 3/12, B05B 13/02

(54) **METHOD FOR COATING OBJECTS**
VERFAHREN ZUR BESCHICHTUNG VON OBJEKTEN
PROCÉDÉ DE REVÊTEMENT D'OBJETS

(30) Priority: 14.10.2008 US 287740
(43) Date of publication of application: 27.07.2011
(73) Proprietor: Bayer Healthcare Llc, Morristown, NJ 07962-1910 (US)
(72) Inventor: BOHLMANN, Ulf, 1071 Auckland (NZ); SCHMITZ, Guido, Sparta NJ 07871 (US); WALTER, Reinhard, Morristown NJ 07962-1910 (US)
(74) Representative: BIP Patents
(86) International application number: PCT/US2009/005612
(87) International publication number: WO 2010/044857

(56) References cited:
- EP-A1- 0 088 317
- WO-A1-2006/117046
- DE-A1- 19 838 540
- US-A1- 2006 002 986
- US-B2- 6 689 417

## Description

### 1. FIELD OF THE INVENTION

This invention relates to methods for coating objects, selected from the group of tablets, caplets, cores for gelcaps, and hard and soft gelatin capsules that require removal of dust after formation. The invention also relates to devices for making such coated objects.

### 2. BACKGROUND OF THE INVENTION

Compressed tablets have long been known and used in the pharmaceutical industry. Tablets are usually made by compressing a powder or granulation by direct compression in a tablet press, although molded tablets are also known. Tablets can be made in many different shapes, and, if the shape is sufficiently elongated, the tablet may be referred to as a caplet. Gelcaps and geltabs are typically formed by dipping a compressed and, optionally, coated caplet or tablet, respectively, in a liquid gelatin bath or by enrobing a caplet between formable sheets of gelatin that are molded around the caplet or tablet. Hard gelatin capsules are typically formed by filling a hard gelatin shell having an open end with a powder, caplet, or granulation containing one or more active ingredients and placing another hard gelatin shell over the open end of the first shell, to close the capsule. Soft gelatin capsules may be made by enrobing a liquid in gelatin. All of these techniques are well known to those skilled in the art of medicament manufacturing.

These formation techniques, especially those that involve powders or granulations, such as conventional tablet presses, often leave excess powder or granules adhering to the surface of the formed object. Tablets and caplets formed in tablet presses, especially, can be vulnerable to dust from left-over powder that adheres to the surface of the tablet or caplet. This dust may interfere with subsequent manufacturing operations, including gelcap formation, and may reduce the efficiency of the manufacturing process. In addition, imperfection in the fit between the press punches and dies of a tablet press may form burrs around the edge of tablets and caplets. These burrs can adversely affect subsequent coating operations. Gelcaps and capsules may present similar problems even after surrounding the powder or caplet with gelatin. Dust from the formation of the caplet or the ambient presence of powder dust resulting from carrying out the capsule filling process in proximity to subsequent coating operations, or even ambient dust, may interfere with subsequent coating or printing operations and may provide undesired contaminants or may reduce the functional or aesthetic features of the finished product.

The problem of dust contamination is not limited to pharmaceutical operations. Many products formed by compression techniques, such as injection molding and other extrusion techniques, and objects that are coated with a powder or another particulate solid material during processing, such as confectionary products, and even large items, like preformed metallic objects that are painted using powder coating techniques can suffer from dust contamination.

Dust control may be accomplished by using large dust filtration system when powder coating large metal objects. These systems are designed to remove dust from the air in the environs of the object, usually where a worker is also present. Such systems, however, do not generally remove the dust from the surface of the metallic object and are directed more toward environmental protection for the worker.

Removing dust from smaller objects, such as pharmaceutical tablets, presents different problems. Dust control is more concerned with removal of residual dust from the surface of tablets and smoothing out residual burrs and irregularities from the formation process than with removal of dust from the air. For tablets and caplets, a dedusting procedure normally follows after the forming the tablet or caplet by compression. A dedusting process step also typically follows a capsule filling operation. Another very common place to include an additional dedusting operation is immediately before a coating operation to ensure that dust, from whatever source, does not interfere with the coating operation.

Dedusting processes are typically carried out in a processing machine called a deduster. Different types of tablet dedusters are known in the art: vertical vibratory dedusters, horizontal vibratory dedusters, and brush type dedusters. Each type has its advantages and disadvantages and its preferred uses.

A vertical vibratory deduster conveys tablets upward or downward through a perforated, spiral channel by vibrating the channel. The vibrations shake off the dust, and the tablets rub and jostle against one another in the channel, thereby removing any burrs and knocking off dust that may be adhering to the tablets. Dedusters are often set up in an enclosed environment so that forced air supplied to the enclosed environment can also blow dust off the tablets and keep the dust airborne. A vacuum system that pulls air from the enclosed environment can also remove dust from the immediate environment of the tablets. Some commercial dedusters have both a forced air blower and a vacuum system operating together.

Horizontal vibratory dedusters have a flat, perforated vibratory bed, rather than a vertical, spiral channel. The bed vibrates and shakes the dust off. Air may also be employed to blow the dust off the the tablets, and, as with a vertical deduster, a vacuum pump may also be employed to remove dust. Unlike vertical units, however, horizontal dedusters are not as effective at moving tablets along the bed. Filling large bins or feeding to high discharge ports requires an additional conveyor or other device to remove the tablets from the deduster. A flat bed, however, is more convenient to clean than a spiral channel and may be a more preferred option for batch operations.

Brush-type tablet dedusters are available in vertical and horizontal configurations. A motor driven rotary brush simultaneously conveys the tablets around the bed or along the channel and physically "wipes" the tablets or capsules to dedust them. Brush-type units have proven to be more suitable for handling capsules, gelcaps and other smooth objects, while ordinary vertical and horizontal dedusters are generally preferred for tablets and other objects with rougher surfaces, where greater friction allows for more efficient movement and turning of the tablets through vibration of the bed.

After objects have been compressed and dedusted, they may be subject to additional production steps. One such additional step may be coating. Coatings for objects such as tablets and other forms are well known, and such coatings may include sugar coating, film coating, enteric coating, gelatin, film forming material, and controlled release coatings. These coatings are often put on tablets by means of a spray coater. In a spray coater, tablets are placed in a receptacle, such as a coating pan, and sprayed with a liquid or aerosol coating material. The pan can be agitated to turn the tablets or other mechanical means can be employed to turn the tablets so that all sides of the tablets are exposed to the coating spray. The tablets are usually exposed to the spray for a predetermined time, and the time of exposure is related to the thickness of the applied coat. The coated tablets are then dried and sent for further processing. Objects other than tablets can be subjected to similar processes, with similar results.

One known method for coating small objects like pharmaceutical tablets is continuous coating. The first continuous coaters were reportedly used to coat seeds for agricultural use. These continuous coaters reportedly had two stacked, perforated cylinders and multiple spray guns. Newer continuous coaters feed tablets into one end of a long, continuous, perforated, rotating cylinder. Tablets are moved through the rotating cylinder by baffles. The tablets are sprayed and dried simultaneously with hot forced air.

A two-step process of dedusting objects in a deduster followed by coating in a continuous coater, or a three-step process in which objects are dried after coating in a separate dryer, is a time consuming process and can lead to inefficiencies in a manufacturing line. For most spray processes, some drying is required, so most processes require three steps. Cleaning cycles for the equipment involved in each process step can mean increased down time in the product line or excess costs associated with extra equipment. Especially in the manufacture of medicaments for human consumption, limiting the entire manufacturing line to the slowest or most frequently shut-down piece of equipment can delay an entire manufacturing run because of the time-consuming process involved in documenting and testing batches of materials and maintaining the records of the cleaning activities. In addition, if three separate process steps are vulnerable to shutting down, then the process is vulnerable to extra cost and delay three different ways.

The documents EP 0 088 317 A1 and DE 198 38 540 A1 disclose dedusting and coating methods and corresponding apparatuses as already successfully employed in the field of coating compressed tablets.

### SUMMARY OF THE INVENTION

The principal object of the invention, therefore, is to provide a method for simultaneously dedusting and coating an object, such as a pharmaceutical tablet.

Another object of the invention is to provide an object, such as a pharmaceutical tablet, that has been simultaneously dedusted and coated.

A further object of the invention is to provide method for dedusting, coating and drying an object, such as a pharmaceutical tablet, in a single process step, with a single piece of process equipment, to improve efficiency and reliability in the process.

Another object of the invention is to provide an object that has been dedusted, coated and dried in a single process step.

To achieve the foregoing objects and in accordance with the purpose of the invention, as embodied and broadly described herein, the invention provides a process for dedusting and coating an object comprising the steps of forming an object subject to contamination by dust; and dedusting the object while simultaneously coating the object to form a finished product. In an alternative embodiment of the invention, the finished product may also be dried in the same process step as the dedusting and coating step.

Further disclosed is an object made by a process comprising the steps of forming an object subject to contamination by dust; and dedusting the object while substantially simultaneously coating the object to form a finished product.

The invention further provides an apparatus for coating and dedusting an object comprising a deduster comprising a dedusting region in which an object resides during dedusting and a coating apparatus aligned to substantially coat the object with a material while said object is in the dedusting region.

The invention further provides a process for dedusting and coating an object comprising the steps of forming an object subject to contamination by dust; and dedusting the object while simultaneously coating the object to form a finished product, which is then dried in a single process step. The object may be dried using an infrared process, such as drying by exposing the objects to infrared emitters.

One advantage of the invention is that it can be made through modification of existing dedusting equipment, including vertical, horizontal and brush dedusters, which reduces the expense of constructing an entirely new apparatus from the ground up.

Another advantage of the invention is that many commercial dedusting devices have vacuum systems that remove ambient dust from the area surrounding the dedusting equipment. In combination with a forced air feed, such a vacuum system can provide a simultaneous drying system for coated materials. Another advantage of the invention is that an infrared drying process may be used either alone or in conjunction with a vacuum or forced air system, to provide improved drying and additional cost savings.

The invention also requires significantly less space than existing batch and continuous coating and dedusting equipment and therefore can be coupled directly to machinery used to form objects. Inventory and material handling costs can thereby be reduced.

In addition the invention provides the opportunity to apply different spray coats either sequentially or at the same time during the coating process.

Yet another advantage of the invention is that most dedusting systems employ a vibration system to shake dust off tablets or other objects. This vibration system can advantageously be used to turn the tablets or other objects so that more or all of the surface area of the object is exposed to the coating medium.

Additional objects and advantages of the invention will be set forth in part in the description that follows, and in part will be apparent from this description, or may be learned by practice of the invention. The objects and advantages of the invention may be realized and attained by means of the instrumentalities and combinations particularly pointed out in the appended claims.

### BRIEF DESCRIPTION OF THE DRAWINGS

Figure 1 shows a drawing of an embodiment of the device in the form of a laboratory-scale vertical deduster that substantially simultaneously dedusts and coats a pharmaceutical tablet.
Figure 2 shows a stripper for getting an even tablet bed depth in the deduster
Figure 3 shows a closer view of Figure 1 that shows a spray nozzle mounted on a frame (not shown) of a vertical deduster and ready to spray tablets lying in a channel of the vertical deduster.
Figure 4 shows an alternative embodiment of the invention in which tablets are exposed to a substantially simultaneous dedusting and coating process and then to a second dedusting process.
Figure 5 shows an infrared emitter employed along the outer rim of the channel of the deduster to facilitate drying of the coated tablets.

### DETAILED DESCRIPTION OF THE PREFERRED EMBODIMENTS

Reference will now be made in detail to the presently preferred embodiments of the invention.

The invention comprises methods for coating compressed tablets, caplets, cores for gelcaps, and hard and soft gelatin capsules that require removal of dust after formation. Moreover, an object may be formed in many different ways. As used herein, the term "forming" should be understood to include any process step that molds, shapes, assembles or otherwise creates an object in one or a plurality of steps and that results in an object subject to potential dust contamination. Compression molding, extrusion, pre-coating with a powder-based or particulate material, a cutting, sanding or polishing step or even manual assembly may provide both the object formed and residual dust on the object itself or in the air around the object, and the object may be said to be contaminated by the dust. "Contaminate" as used herein is intended to encompass chemical contamination or adulteration and contamination in the sense that the presence of dust on the object could interfere with equipment or processes carried out in later manufacturing operations, including coating, and also in the sense that the presence of dust could interfere with the physical or aesthetic properties desired for the object from manufacturing steps like coating. "Contaminated" objects with excess dust on their surfaces can lead to increased rates of rejection of completed objects associated with increased cost per unit for the successfully manufactured objects as the manufacturing costs must be spread over fewer successfully processed objects.

The invention also relates to objects formed by such methods and to devices for making such coated objects.

The embodiments set forth herein in detail also demonstrate the use of the invention with a vertical deduster. Those skilled in the art will appreciate that the invention may be employed with other types of dedusters as well. Horizontal dedusters and brush type dedusters may be advantageously employed with the invention. Spray coaters may be directed towards the tray or channel of a horizontal or brush type deduster, and the advantages of the invention may be realized. The selection of which type of deduster to use in a given application depends on factors beyond the invention itself, such as the identity and amount of the object to be treated. Drying equipment as discussed herein may also be directed toward the channel or tray of these alternative dedusters with similar advantageous results.

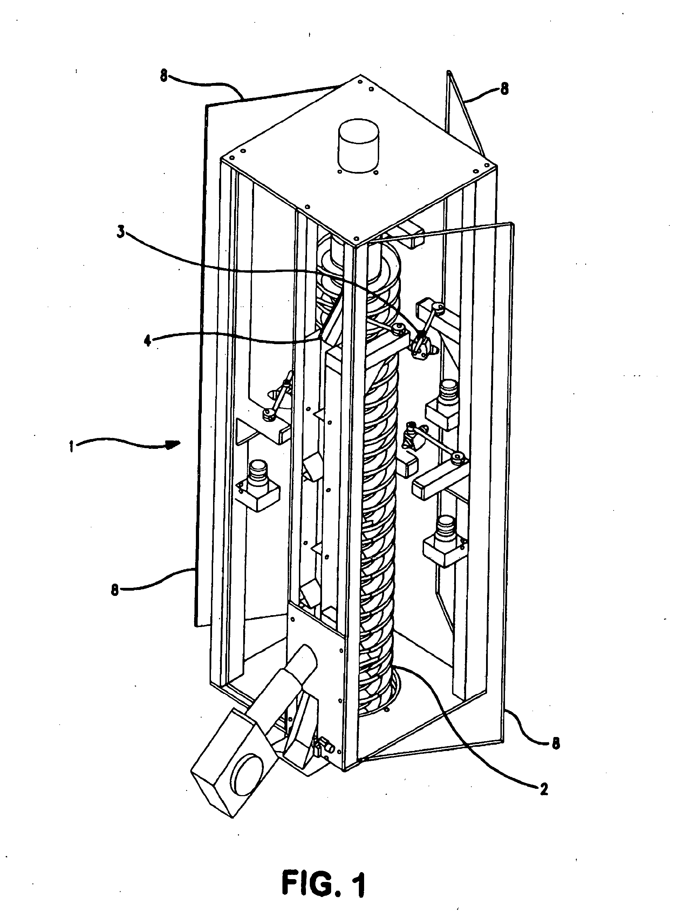

In Fig. 1, newly pressed tablets are discharged from a tablet press (not shown in Fig. 1) and fed into a combination tablet deduster/coater 1. The coater/deduster moves the tablets spirally upward through a perforated channel 2 using a vibration system known in the art. As the tablets move up the channel, they are exposed to a fluid or particulate coating material that is sprayed through a nozzle 3. The number of nozzles used to achieve the desired coating composition and average weight gain or thickness may be adjusted as desired by one skilled in the art. The positioning of the nozzle along the channel, also measured by the height of the nozzle in a vertical deduster/coater is set high enough so that sufficient dedusting has taken place in the lower region of the channel to allow effective coating and low enough so that the tablet coating has dried by the desired amount by the time the tablets pass through the exit ramp 4 for further processing. An advantage of the invention is that vibration provides random tumbling of the tablets, at least when the number of tablets in the channel allows tumbling, which allows exposure of all tablet surfaces to the spray material, thereby assisting in providing a thorough coating of the tablet. The installation of baffles in the channel (not shown) can further facilitate random tumbling of the tablets. Design and placement of the baffles may be adjusted according to the desires of operator to achieve a consistent coat on all objects.

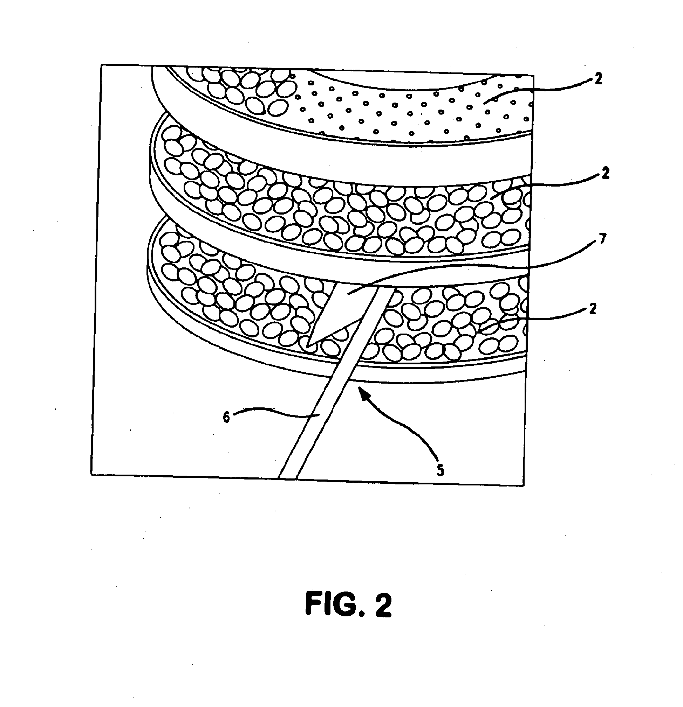

In the lower zone of the deduster, identified as the dedusting zone, a stripper may be installed to assure a defined constant tablet bed depth in the channel. Figure 2 shows a stripper 5 comprising a fixed arm 6 and a downwardly directed flexible curtain 7 that evens the depth of the tablets across the channel 2. Any tablets stripped from the bed by stripper 5 fall down into the inlet reservoir of the deduster for subsequent processing through the deduster.

The equipment of the invention may be realized by modification of existing commercial dedusters. A commercial deduster may be adapted for use in the invention by the addition of one or more nozzles and optional drying equipment. As a result, the characteristics of the deduster are typically dictated by the particular process needs of the manufacturing operation and the characteristics of a commercial deduster, which are fixed by the manufacturer of the deduster.

Nozzle design and placement in the deduster are dictated by the needs of the process. The nozzle should be set a sufficient height above the entrance point of the objects so that at least some dedusting has taken place before spraying commences. Otherwise, the channel holes or the channel itself may become clogged with an agglomeration of dust and coating material, which could degrade the uniformity of the coating process and require additional down time in the manufacturing line for equipment cleaning. The nozzle should also not be set so high in the device that the coated objects are still too tacky or too wet for further processing when they leave the deduster/coater. While the invention contemplates drying operations for wet objects that are situated inside the deduster/coater or that comprise a separate process step, faster drying times are generally preferred, at least in tablet manufacturing, because tacky tablets tend to adhere to each other, and a coat can easily lose its desirable finish if processed when wet.

The nozzle (or nozzles) should also be positioned to deliver the maximum amount of spray to the object to be coated and to minimize the amount of wasted coating material. The atomizing function and the air pattern of the spray nozzle should not have a negative effect of the movement of the objects in and along the channel.

Those skilled in the art will recognize that the term "simultaneous" when used with dedusting and coating means that the processes are carried out within a single piece of equipment rather than in two distinct pieces of equipment requiring transport of tablets from one piece of equipment to the other. Even if preliminary or subsequent dedusting takes place before or after the coating process, dedusting and coating are "simultaneous" if one deduster in a series of dedusters is equipped with a coating apparatus and that apparatus is used for its dedusting function and for coating objects in the same operation. "Simultaneous" as used with this invention does not require exact coincidence in time.

The deduster/coater shown in Fig. 1 also includes a defined region which serves as a housing, comprising a plurality of panels 8, which may be transparent. These panels may be closed during operation to assist in the dedusting process by increasing the effectiveness of a circulating air/vacuum system used in dedusting, to limit introduction of foreign materials from the ambient air of the factory floor, and to limit the escape of aerosols and other airborne materials resulting from a spray coating operation.

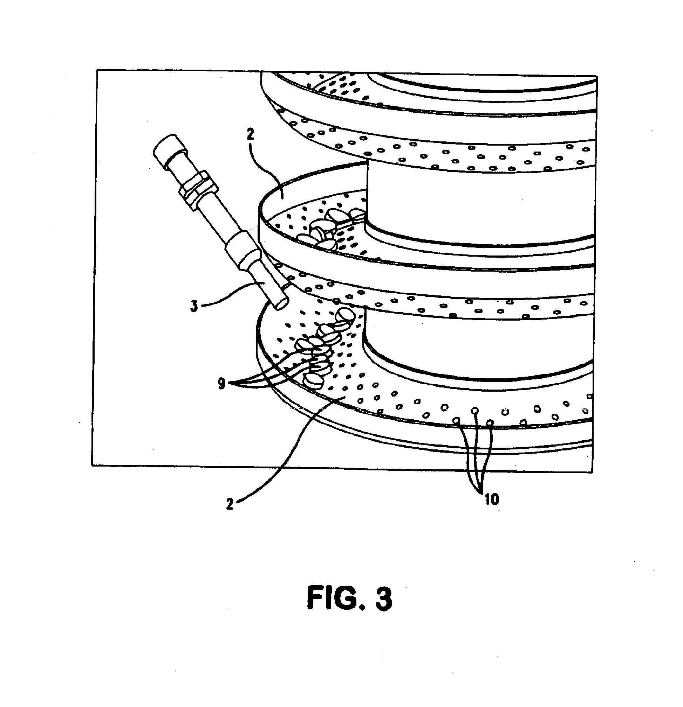

The spray nozzle 3 is shown in more detail in Fig. 3. Tablets 9 proceed up the spiral channel 2. The channel has perforations 10 that allow for removal of dust and assist in air flow through and around tablets to increase the rate at which the dedusting occurs and the rate at which the coating material dries. Additional slots in the body of the spiral channel (not shown) are generally required for purging the evaporated gas generated by a spray coating operation immediately after the spray coating operation takes place.

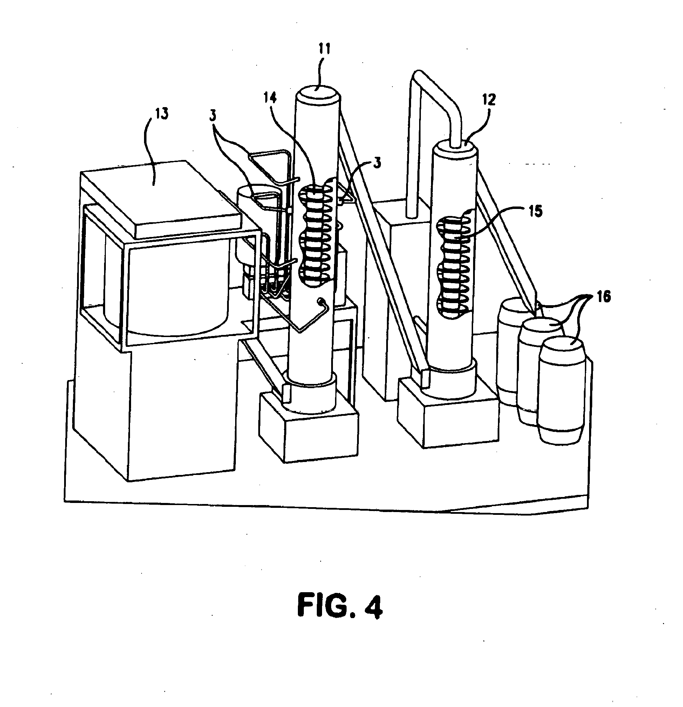

An alternative embodiment of the invention is shown in Fig. 4. A plurality of dedusters 11, 12 is arranged in series. Tablets emerge from tablet press 13 and proceed up the respective perforated spiral channels 14, 15 in the dedusters. A plurality of nozzles 3 is arrayed on the first deduster 11 and the coated tablets are further dried in the second deduster 12. The coated tablets are then transferred to drums 16 for further processing or packaging.

A plurality of nozzles may be used to deliver more of a single coating or may be arranged to deliver a second coat of the coating material or may be arranged to provide series or parallel administrations of two different materials for coating. Thus, a spray coating comprising two separate formulations may be simultaneously applied, permitting combinations of otherwise incompatible materials or even chemical reaction between ingredients of the two coating formulations while drying on the coated object.

Vertical dedusters/coaters are preferred in the invention, because of their large throughput (up to about 600,000 tablets per hour), but, as discussed above, horizontal dedusters may also be used with one or more spray nozzles disposed over the perforated vibratory bed. Brush type dedusters may be used, but are not as highly preferred, because the brush may mar the coating of the tablets if the coated tablets are brushed when wet, so care must be taken to ensure that nozzles are positioned downstream from the brushes if specific appearance standards, incompatible with brushing wet objects, is required. Also, the brushes may become clogged with coating material, which could interfere with dedusting efficiency and could entail additional cleaning operations.

The coating material may be any material or materials used for coating an object. Preferred coating materials when the objects to be coated comprise tablets, pharmaceutical delivery vehicles such as caplets and capsules and other objects for human consumption like confections, include any coating materials conventionally used in tablet coating. Typical coating materials include sugar solutions, polymers, polysaccharides and other known film forming agents. These materials may also include plasticizers, pigments and other materials. One advantage of the invention is that different coating materials may be supplied to different nozzles in the production line, allowing for multiple coats or several different coating materials.

As shown in Fig. 4, multiple dedusters may be used. Also contemplated by the invention are systems in which multiple dedusters with additional nozzles are used. Tablets may be dedusted and coated in a first deduster/coater and then either a second coat of the same material or a coating of a different material may be applied in a second deduster/coater or, as discussed above, in sequential or identical regions of the same deduster/coater.

In a preferred embodiment of the invention, where the objects being formed are pharmaceutical tablets, the apparatus has a throughput of from about 500 tablets to about 10,000 tablets per minute, depending on the needs of the operator, the tablet shape, tablet size and operating speed of the tablet press. Depending on the type of object being formed, a buffer tank (not shown) may preferably be fixed between the outlet of the forming equipment, such as a tablet press, and the inlet of the deduster/coater of the invention to provide a more constant flow of objects, such as tablets, into the deduster/coater. Typical transit times in the deduster/coater for a typical tablet is preferably from about 3 to about 20 minutes to complete a 1-2 % weight gain for tablets having a non-functional, decorative coating.

Preferably, when tablets are used, the tablets are either still warm from the compression process or have been preheated to a temperature of from about 40 °C to about 80 °C before entering the spraying zone. The spraying zone can be divided in different segments dependent on the number of nozzles required for the process. Depending on the desired tablet weight gain, tablet shape, or desired effect, the spraying zone may be equipped with multiple segments and spray nozzles to achieve the desired result.

Since it is very difficult to measure the thickness of tablet coatings, it is customary in the industry to measure coating by calculating the percent weight gain of tablets during the coating process. In operation of the invention, round 600 mg tablets with a target coating weight gain of 1% are exposed to from about 3 to about 6 segments or spraying nozzles in the deduster/coater. If appropriate, the number of nozzles can be increased in the equipment, a second coating may be applied, or the tablets can recirculate within the unit to obtain the desired coating thickness. As shown in Figure 4, a second deduster/coater unit may optionally be connected to a first unit, to extend the exposure of tablets to the spraying process or to dedust the coated tablets, if the spraying process introduces additional dust contamination.

Each nozzle can be independently controlled to obtain preferred spray patterns using the same or different coating solutions. Multilayer coatings may provide different colors or different functions in the coating materials.

The spray rate per nozzle depends on the number of tablets being conveyed and the general process set-up. Preferred spray rates per nozzle range from about 1 g to about 100 g of spray solution applied per minute. The temperature in each spraying zone can be set to any desired temperature level. For non-functional decorative coatings, a preferred temperature range is from about 60 °C to about 100 °C.

When the deduster is a preferred vertical deduster with an air source to assist in dedusting and drying, the preferred air volume through the equipment is about 200 m³/h for the dedusting step and from about 200 m³/h to about 500 m³/h for purging the evaporated gas out of the equipment. The air flow for dedusting typically flows downwards in commercial vertical dedusters, and the air flow for purging gas from the spray-coating process in accordance with the invention should generally flow upwards. Thus, the air streams may preferably be separated in an apparatus in accordance with the invention.

After leaving the spraying zone the tablets may be further dried and cooled while still in the deduster/coater, or the tablets may be sent for further drying in a subsequent deduster, as discussed above. In a preferred embodiment of the invention, however, the tablets are dried in the deduster using either a forced air or vacuum system or a heating system or a combination of the two systems.

Commercial-scale tablet coaters, like batch or continuous coaters, use a large volume (anywhere from about 1,000 to about 10,000 m³/h) of dehumidified hot air. This volume of heated, dehumidified air can require a substantial amount of expensive energy and process equipment to create and maintain.

In a preferred embodiment of the invention, one or more infrared (IR) emitters are incorporated into the deduster system of the invention. If a sufficient number of IR emitters are utilized, no substantial additional airflow is required other than a ventilator for purging evaporated water from the coating step.

Emitters for supporting the process with the required energy for the drying step may be installed in different positions in the deduster. By using different IR emitter sections, drying characteristics of the tablets or coated objects may be controlled and modified for various purposes.

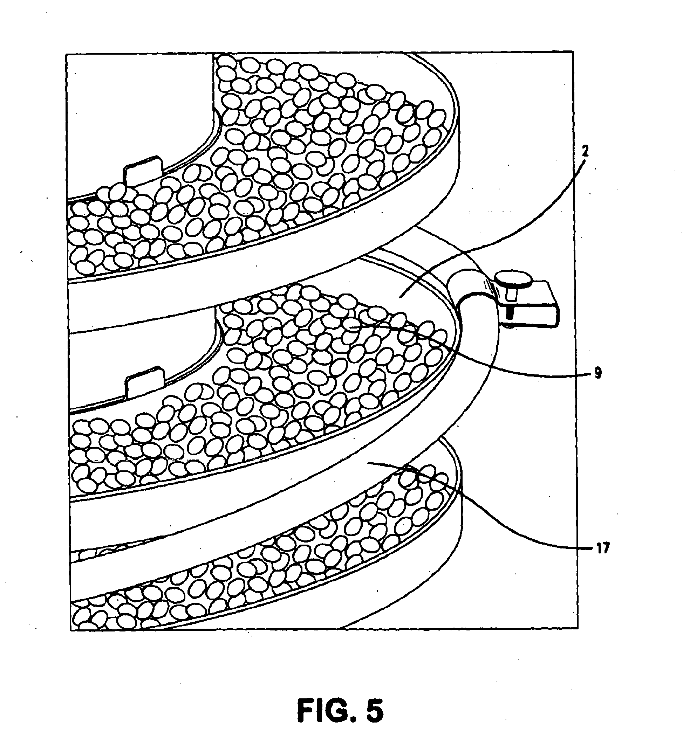

An IR emitter of the invention is shown in more detail in Figure 5. The laboratory-scale deduster of Figure 1 is shown in close-up. A channel 2 holds tablets 9 that have recently been coated in accordance with the invention. An IR emitter 17 is mounted along the periphery of the channel 2, providing heat to the coated tablets 9 in the channel. The amount of heat supplied by each IR emitter will depend on many different factors, including the throughput of tablets, the amount of coating material to be dried, the coating formulation and the number of IR emitters used in the process step.

## Claims

1. A process for coating objects selected from the group consisting of compressed tablets, caplets, cores for gelcaps, and hard and soft gelatin capsules comprising the steps of:
a. forming objects subject to contamination by dust;
b. placing the objects having adhered dust particles in a perforated channel or bed to remove said adhered dust, said perforated channel or bed having an entrance point, where the objects are fed into the perforated channel or bed, and an exit point, where the objects leave the perforated channel or bed;
c. removing at least part of said adhered dust by vibrating the objects by means of a vibratory system to shake dust free from the objects while moving the objects from the entrance point to the exit point of the perforated channel or bed; and
d. coating said objects while said objects are within the perforated channel or bed in a spraying zone by means of a coating apparatus comprising one or more spray nozzles which define the spraying zone.

2. The process of claim 1, wherein said objects are tablets.

3. The process of claim 2, wherein said step of forming is carried out in a tablet press.

4. The process of claim 1, further comprising the step of drying said objects after coating said objects.

5. The process of claim 4, wherein said drying of said objects takes place while they are within a drying zone located downstream of the spraying zone between the entrance point and the exit point.

6. The process of claim 4, wherein said step of drying said objects comprises the step of exposing said objects to radiant energy for a time sufficient to substantially dry said objects, said radiant energy preferably comprising infrared energy.

7. An apparatus for coating objects selected from the group consisting of compressed tablets, caplets, cores for gelcaps, and hard and soft gelatin capsules comprising:
a. a perforated channel or bed in which objects having adhered dust particles are to be placed during a process to remove said adhered dust, said perforated channel or bed having an entrance point, where the objects are fed into the perforated channel or bed, and an exit point, where the objects leave the perforated channel or bed;
b. a device for removing at least part of said adhered dust comprising a vibratory system for vibrating the objects to shake dust free from the objects and to move the objects from the entrance point to the exit point of the perforated channel or bed; and
c. a coating apparatus comprising one or more spray nozzles for coating said objects while said objects are within the perforated channel or bed; and
d. between entrance point and exit point of the perforated channel or bed a spraying zone defined by the spray nozzles.

8. The apparatus of claim 7, wherein said device for removing at least part of said adhered dust comprises a vertical deduster.

9. The apparatus of claim 7 further comprising a drying mechanism for drying said coated objects while said objects are within said perforated channel or bed.

10. The apparatus of claim 7 further comprising a drying zone located downstream of the spraying zone and a drying mechanism for drying said coated objects while said objects are within said drying zone.

11. The apparatus of claim 9 or 10, wherein said drying mechanism comprises at least one infrared emitter.

12. The apparatus of claim 7 further comprising baffles installed in the perforated channel or bed.

13. The apparatus of claim 7, further comprising a stripper installed above the perforated channel or bed for evening the depth of objects across the perforated channel or bed.

## Patentansprüche

1. Verfahren zum Beschichten von Objekten ausgewählt aus der Gruppe bestehend aus komprimierten Tabletten, Caplets, Kernen für Gelcaps und harten und weichen Gelatinekapseln, umfassend die Schritte:
a. Formgebung von Objekten, die Kontamination durch Staub unterliegen;
b. Anordnen der Objekte mit anhaftenden Staubpartikeln in einem perforierten Kanal oder Bett, um den anhaftenden Staub zu entfernen, wobei der/das perforierte Kanal oder Bett einen Einlasspunkt aufweist, an dem die Objekte dem perforierten Kanal oder Bett zugeführt werden, und einen Austrittspunkt, an dem die Objekte den perforierten Kanal oder das perforierte Bett verlassen;
c. Entfernen wenigstens eines Teils des anhaftenden Staubs durch Vibrieren der Objekte mithilfe eines Vibrationssystems, um Staub von den Objekten freizuschütteln, während die Objekte von dem Einlasspunkt zu dem Austrittspunkt des perforierten Kanals oder Betts bewegt werden; und
d. Beschichten der Objekte, während sich die Objekte innerhalb des perforierten Kanals oder Betts befinden, in einer Sprühzone mithilfe einer Beschichtungsvorrichtung, die eine oder mehrere Sprühdüsen, die die Sprühzone definieren, umfasst.

2. Verfahren gemäß Anspruch 1, wobei die Objekte Tabletten sind.

3. Verfahren gemäß Anspruch 2, wobei der Schritt der Formgebung in einer Tablettenpresse durchgeführt wird.

4. Verfahren gemäß Anspruch 1, ferner umfassend den Schritt des Trocknens der Objekte nach dem Beschichten der Objekte.

5. Verfahren gemäß Anspruch 4, wobei das Trocknen der Objekte erfolgt, während sie sich in einer Trocknungszone befinden, die der Sprühzone nachgeschaltet zwischen dem Einlasspunkt und dem Austrittspunkt angeordnet ist.

6. Verfahren gemäß Anspruch 4, wobei der Schritt des Trocknens der Objekte den Schritt des Exponierens der Objekte gegenüber Strahlungsenergie über eine Zeit umfasst, die ausreicht, um die Objekte wesentlich zu trocknen, wobei die Strahlungsenergie vorzugsweise Infrarotenergie umfasst.

7. Vorrichtung zum Beschichten von Objekten ausgewählt aus der Gruppe bestehend aus komprimierten Tabletten, Caplets, Kernen für Gelcaps und harten und weichen Gelatinekapseln, umfassend:
a. einen perforierten Kanal oder ein perforiertes Bett, worin Objekte mit anhaftenden Staubpartikeln während eines Verfahrens zum Entfernen des anhaftenden Staubs angeordnet werden sollen, wobei der/das perforierte Kanal oder Bett einen Einlasspunkt aufweist, an dem die Objekte dem perforierten Kanal oder Bett zugeführt werden, und einen Austrittspunkt, an dem die Objekte den perforierten Kanal oder das perforierte Bett verlassen;
b. eine Vorrichtung zum Entfernen wenigstens eines Teils des anhaftenden Staubs, umfassend ein Vibrationssystem zum Vibrieren der Objekte, um Staub von den Objekten freizuschütteln und die Objekte von dem Einlasspunkt zu dem Austrittspunkt des perforierten Kanals oder Betts zu bewegen; und
c. eine Beschichtungsvorrichtung, die eine oder mehrere Sprühdüsen umfasst, zum Beschichten der Objekte, während sich die Objekte in dem perforierten Kanal oder Bett befinden; und
d. eine durch die Sprühdüsen definierte Sprühzone zwischen dem Einlasspunkt und dem Austrittspunkt des perforierten Kanals oder Betts.

8. Vorrichtung gemäß Anspruch 7, wobei die Vorrichtung zum Entfernen wenigstens eines Teils des anhaftenden Staubs einen Vertikalentstauber umfasst.

9. Vorrichtung gemäß Anspruch 7, ferner umfassend einen Trocknungsmechanismus zum Trocknen der beschichteten Objekte, während sich die Objekte in dem perforierten Kanal oder Bett befinden.

10. Vorrichtung gemäß Anspruch 7, ferner umfassend eine der Sprühzone nachgeschaltet angeordnete Trocknungszone und einen Trocknungsmechanismus zum Trocknen der beschichteten Objekte, während sich die Objekte in der Trocknungszone befinden.

11. Vorrichtung gemäß Anspruch 9 oder 10, wobei der Trocknungsmechanismus wenigstens einen Infrarotstrahler umfasst.

12. Vorrichtung gemäß Anspruch 7, ferner umfassend in dem perforierten Kanal oder Bett angeordnete Blenden.

13. Vorrichtung gemäß Anspruch 7, ferner umfassend einen über dem perforierten Kanal oder Bett angeordneten Abstreifer zum Einebnen der Tiefe der Objekte über den perforierten Kanal oder das perforierte Bett.

## Revendications

1. Procédé pour revêtir des objets sélectionnés dans le groupe comprenant des pastilles compressées, des comprimés, des coeurs de gélules et des capsules de gélatine dures et molles, comprenant les étapes suivantes:
a. former des objets sensibles à une contamination par la poussière;
b. placer les objets comportant des particules de poussière collées dans un canal ou un lit perforé dans le but d'enlever ladite poussière collée, ledit canal ou lit perforé présentant un point d'entrée, à travers lequel les objets sont amenés dans le canal ou le lit perforé, et un point de sortie, à travers lequel les objets quittent le canal ou le lit perforé;
c. enlever au moins une partie de ladite poussière collée en faisant vibrer les objets au moyen d'un système de vibration afin de décoller la poussière des objets tout en déplaçant les objets à partir du point d'entrée jusqu'au point de sortie du canal ou du lit perforé; et
d. revêtir lesdits objets pendant que lesdits objets se trouvent à l'intérieur du canal ou du lit perforé dans une zone de pulvérisation au moyen d'un dispositif de revêtement comprenant une ou plusieurs buse(s) de pulvérisation qui définissent la zone de pulvérisation.

2. Procédé selon la revendication 1, dans lequel lesdits objets sont des comprimés.

3. Procédé selon la revendication 2, dans lequel ladite étape de formage est exécutée dans une presse à comprimés.

4. Procédé selon la revendication 1, comprenant en outre l'étape de séchage desdits objets après le revêtement desdits objets.

5. Procédé selon la revendication 4, dans lequel ledit séchage desdits objets se produit pendant que ceux-ci se trouvent à l'intérieur d'une zone de séchage qui est située en aval de la zone de pulvérisation entre le point d'entrée et le point de sortie.

6. Procédé selon la revendication 4, dans lequel l'étape de séchage desdits objets comprend l'étape d'exposition desdits objets à une énergie rayonnante pendant une période de temps suffisante pour sécher sensiblement lesdits objets, ladite énergie rayonnante comprenant de préférence une énergie infrarouge.

7. Dispositif pour revêtir des objets sélectionnés dans le groupe comprenant des pastilles compressées, des comprimés, des coeurs de gélules et des capsules de gélatine dures et molle, comprenant:
a. un canal ou un lit perforé dans lequel des objets comportant des particules de poussière collées doivent être placés pendant un procédé pour enlever ladite poussière collée, ledit canal ou lit perforé présentant un point d'entrée, à travers lequel les objets sont amenés dans le canal ou le lit perforé, et un point de sortie, à travers lequel les objets quittent le canal ou le lit perforé;
b. un dispositif pour enlever au moins une partie de ladite poussière collée, comprenant un système de vibration pour faire vibrer les objets dans le but de décoller la poussière des objets et de déplacer les objets à partir du point d'entrée jusqu'au point de sortie du canal ou du lit perforé; et
c. un dispositif de revêtement comprenant une ou plusieurs buse(s) de pulvérisation pour revêtir lesdits objets pendant que lesdits objets se trouvent à l'intérieur du canal ou du lit perforé; et
d. une zone de pulvérisation définie par les buses de pulvérisation entre le point d'entrée et le point de sortie du canal ou du lit perforé.

8. Dispositif selon la revendication 7, dans lequel ledit dispositif pour enlever au moins une partie de ladite poussière collée comprend un dépoussiéreur vertical.

9. Dispositif selon la revendication 7, comprenant en outre un mécanisme de séchage pour sécher lesdits objets revêtus pendant que lesdits objets se trouvent à l'intérieur dudit canal ou lit perforé.

10. Dispositif selon la revendication 7, comprenant en outre une zone de séchage qui est située en aval de la zone de pulvérisation et un mécanisme de séchage pour sécher lesdits objets revêtus pendant que lesdits objets se trouvent à l'intérieur de la zone de séchage.

11. Dispositif selon la revendication 9 ou 10, dans lequel ledit mécanisme de séchage comprend au moins un émetteur infrarouge.

12. Dispositif selon la revendication 7, comprenant en outre des chicanes qui sont installées dans le canal ou le lit perforé.

13. Dispositif selon la revendication 7, comprenant en outre un rectificateur qui est installé au-dessus du canal ou du lit perforé pour égaliser la profondeur des objets à travers le canal ou le lit perforé.
